(19) Europäisches Patentamt
European Patent Office
Office européen des brevets

(11) Publication number: **0 232 751 B1**

# (12) EUROPEAN PATENT SPECIFICATION

(45) Date of publication of patent specification: **11.09.91**

(51) Int. Cl.⁵: **C07D 273/00**, C07D 257/02, A61K 49/00, A61K 49/04

(21) Application number: **87100635.9**

(22) Date of filing: **19.01.87**

The file contains technical information submitted after the application was filed and not included in this specification

## (54) 1-substituted-4,7,10-triscarboxymethyl-1,4,7,10-tetraazacyclododecane and analogs.

(30) Priority: **23.01.86 US 821725**

(43) Date of publication of application: **19.08.87 Bulletin 87/34**

(45) Publication of the grant of the patent: **11.09.91 Bulletin 91/37**

(84) Designated Contracting States: **BE CH DE FR GB IT LI LU NL SE**

(56) References cited:
**EP-A- 0 071 564**
**EP-A- 0 124 766**
**WO-A-82/04252**
**DE-A- 2 740 170**
**GB-A- 2 108 106**

(73) Proprietor: **E.R. Squibb & Sons, Inc.**
**Lawrenceville-Princeton Road**
**Princeton, N.J. 08540-4000(US)**

(72) Inventor: **Tweedle, Michael F.**
**21 St. David Road**
**Cherry Hill New Jersey(US)**
Inventor: **Gaughan, Glen T.**
**41 Spring Street, Apt. 22**
**Princeton New Jersey(US)**
Inventor: **Hagan, James J.**
**12 Ardmore Place**
**Holmdel New Jersey(US)**

(74) Representative: **Staub, Gabriella, Dr. et al**
**MODIANO, JOSIF, PISANTY & STAUB Via Meravigli, 16**
**I-20123 Milan(IT)**

**EP 0 232 751 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Metal-chelating ligands are useful in diagnostic medicine as contrast agents and relaxation enhancement agents. X-ray imaging, radionuclide imaging, ultrasound imaging and magnetic resonance imaging can each be enhanced by the use of a metal atom bound to a chelating ligand. For example, a chelating ligand can be a radiopharmaceutical when it is prepared as a chelate complex with $^{99m}Tc$, $^{111}In$, $^{67}Ga$, $^{140}La$, $^{169}Yb$ or other radioactive metal ions. When a chelating ligand is complexed with the stable isotopes of the lanthanides, tantalum, bismuth or other elements with molecular weight higher than iodine, the resulting complex absorbs x-rays sufficiently to act as an x-ray contrast agent. In some cases, the agents that are useful in x-ray imaging absorb, reflect or scatter ultrasound radiation sufficiently to be used as an ultrasound agent. If a chelating ligand is complexed with a paramagnetic metal atom that has a symmetric electronic ground state (e.g., $Gd^{+3}$, $Mn^{+2}$ or $Fe^{+3}$) the resulting complex will be useful as a spin relaxation catalyst that is used in magnetic resonance imaging (also known as NMR imaging) as a contrast agent. If a chelating agent is complexed with a paramagnetic metal atom that has an unsymmetrical electronic ground state (e.g., dysprosium (III), holmium (III) and erbium (III)), the resulting complex will be useful as a chemical shift agent in magnetic resonance imaging or in magnetic resonance in vivo spectroscopy. When a chelating agent is complexed with bismuth or tantalum, the resulting complex will be useful as a contrast agent for x-ray imaging.

European Patent Application No. 0 124 766 discloses the symmetrical 1,4,7,10-tetracarboxymethy1-1,4,7,10-tetraazacyclo-dodecane which can be complexed with a paramagnetic metal to form a contrast agent for NMR imaging. European Patent Application No. 0 071 564 discloses cyclic polyamines for NMR imaging containing three substituted ring nitrogens and a ring oxygen or sulfur. PCT Application No. WO 8 204 252 discloses cyclic polyamines, for complexing alkali and alkaline earth metal ions, containing three hydroxyethyl-substituted ring nitrogens and one ring oxygen. UK Patent Application 2 108 106 discloses a class of cyclic polyamines for dissolving barium sulfate scale.

It is an object of this invention to provide new metal-chelating ligands.

It is a further object of this invention to provide metal-chelating ligands whose metal chelate complexes have low osmolality.

It is a further object of this invention to provide metal-chelating ligands whose metal chelate complexes have low acute toxicity.

It is a further object of this invention to provide metal-chelating ligands which, when complexed with a paramagnetic metal atom, have good efficacy as relaxation catalysts in magnetic resonance imaging.

These, and other objects which will be appreciated by the practitioner of this invention, are achieved by metal-chelating ligands having the formula

I

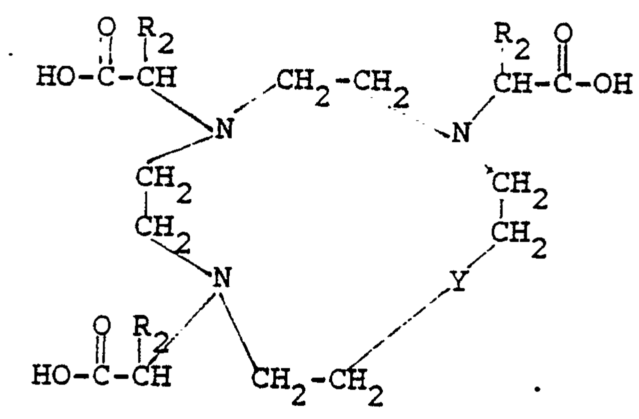

.

In formula I, and throughout the specification, the symbols are as defined below.

Y is

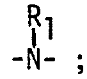

;

$R_1$ is hydrogen, alkyl, arylalkyl, or aryl; and

$R_2$ is hydrogen or alkyl.

The terms "alkyl" and "alkoxy" as used throughout the specification, refer to both straight and branched chain groups having 1 to 5 carbon atoms and methyl is the most preferred alkyl group.

The term "aryl" as used throughout the specification refers to phenyl and substituted phenyl. Substituted phenyl groups are those substituted with 1, 2 or 3 halogen, hydroxyl, alkyl, alkoxy, carbamoyl or carboxyl groups.

The metal-chelating ligands of formula I, and salts thereof, can be complexed with a paramagnetic metal atom and used as relaxation enhancement agents for magnetic resonance imaging. These agents, when administered to a mammalian host (e.g., humans), catalyze relaxation of protons in the body that have been excited by the absorption of radiofrequency energy from a magnetic resonance imager. This acceleration of the rate of relaxation of the excited protons provides for an image of different contrast when the host is scanned with a magnetic resonance imager. The magnetic resonance imager is used to record images at various times before and after administration of the agents, and the differences in the images created by the agents' presence in tissues are used in diagnosis. In proton magnetic resonance imaging, paramagnetic metal atoms such as gadolinium (III), manganese (II), chromium (III), and iron (III) (all are paramagnetic metal atoms with a symmetrical electronic configuration) are preferred as metals complexed by the ligands of formula I; gadolinium (III) is most preferred due to the fact that it has the highest paramagnetism and low toxicity.

The metal-chelating ligands of formula I can be complexed with a lanthanide (atomic number 58) to 71) and used as chemical shift agents in magnetic resonance imaging or in magnetic resonance in vivo spectroscopy.

While the above-described uses for the metal-chelating ligands of formula I are preferred, those working in the diagnostic arts will appreciate that the ligands can also be complexed with the appropriate metals and used as contrast agents in x-ray imaging, radionuclide imaging and ultrasound imaging.

To use the ligands of this invention for imaging, they must first be complexed with the appropriate metal. This can be accomplished by methodology known in the art. For example, the metal can be added to water in the form of an oxide or in the form of a halide and treated with an equimolar amount of a ligand of formula I. The ligand can be added as an aqueous solution. Dilute acid or base can be added (if needed) to maintain a neutral pH. Heating at temperatures as high as 100 °C for periods up to four hours is sometimes required, depending on the metal and the chelator concentration.

Pharmaceutically acceptable salts of the metal complexes of the ligands of this invention are also useful as imaging agents. They can be prepared by using a base (e.g., an alkali metal hydroxide, meglumine or arginine) to neutralize the above-prepared metal complexes while they are still in solution. Some of the metal complexes are formally uncharged and do not need cations as counterions. Such neutral complexes are preferred over charged complexes because they provide solutions of greater physiologic tolerance due to their lower osmolality.

Sterile aqueous solutions of the chelate-complexes can be administered to mammals (e.g., humans) orally, intrathecally and especially intravenously in concentrations of 0.003 to 1.0 molar. For example, for the visualization of brain lesions in canines using magnetic resonance imaging, a gadolinium complex of a ligand of formula I can be administered intravenously at a dose of 0.05 to 0.5 millimoles of the complex per kilogram of animal body weight, preferably at a dose of 0.1 to 0.25 millimole/kilogram. For visualization of the kidneys, the dose is preferably 0.05 to 0.25 millimoles/kilogram. For visualization of the heart, the dose is preferably 0.25 to 1.0 millimoles/kilogram. The pH of the formulation will be between about 6.0 and 8.0, preferably between about 6.5 and 7.5. Physiologically acceptable buffers (e.g., tris(hydroxymethyl)-aminomethane) and other physiologically acceptable additives (e.g., stabilizers such as parabens) can be present.

The compounds of formula I can be prepared by the reaction of a compound having the formula

II

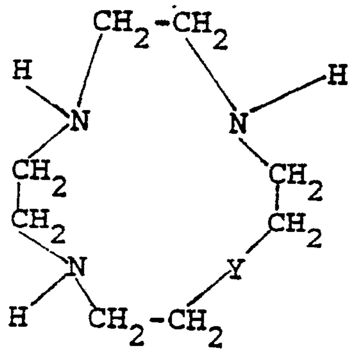

with a reactive acid derivative having the formula

III

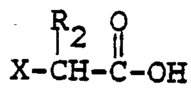

wherein X is a readily displaceable group such as chlorine, bromine or iodine.

In preparing those compounds of formula I wherein Y is

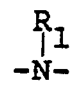

other than -NH-, the above-described reaction of a compound of formula II with a compound of formula III is preferably carried out in water at a pH of about 9 to 10 (most preferably about 9.5). The reaction proceeds most readily if it is warmed to about 50-80 °C. Base, such as an alkali metal hydroxide or a tetraalkylammonium hydroxide, can be used to adjust and maintain the pH of the reaction. After the reaction is completed (usually about 6 to 18 hours) it is allowed to cool (to about 21 °C) and then acidified (using, for example, hydrochloric acid).

In preparing those compounds of formula I wherein Y is -NH-, the above-described reaction of a compound of formula II with a compound of formula III is preferably carried out in water at a pH of about 8.5 to 9 and the temperature of the reaction is maintained at about 45-55 °C. Preferably, only about two equivalents of a compound of formula III are initially used in the reaction; an additional equivalent of the compound of formula III is added in portions starting about 2 to 3 hours after the reaction begins. Total reaction time will preferably be about 8 to 24 hours. The desired trisubstitued product can be separated from the reaction mixture, which includes the mono-, di-, tri- and tetra-substituted derivatives, by art-recognized techniques including selective precipitation, chromatography and crystallization.

Additional synthetic approaches for preparing the compounds of this invention will be apparent to those of ordinary skill in the art. For example, those compounds of formula I wherein Y is

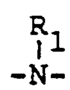

and $R_1$ is alkyl, arylalkyl or aryl can be prepared by alkylation of the corresponding compound of formula I wherein Y is -NH-. Those compounds of formula I wherein Y is -NH- can be prepared by debenzylation of the corresponding compound of formula I wherein Y is

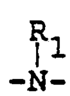

and $R_1$ is benzyl. The debenzylation reaction can be accomplished using catalytic hydrogenation.

Those starting compounds of formula II wherein Y is -NH- are known. The compounds of formula II wherein Y is

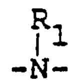

and $R_1$ is alkyl, arylalkyl or aryl (this subgenus is referred to hereinafter as $R'_1$) are novel. The above starting compounds for use in preparing the compounds of formula I constitute an integral part of this invention. They can be prepared from the compound Of formula II wherein Y is -NH- using conventional alkylation techniques.

Alternatively, the starting compounds of formula II wherein Y is

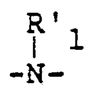

can be prepared by first reacting the tritosylate of diethanolamine with the 1,7-ditosylate of a 4-substituted 1,4,7-triazaheptane to yield

IV

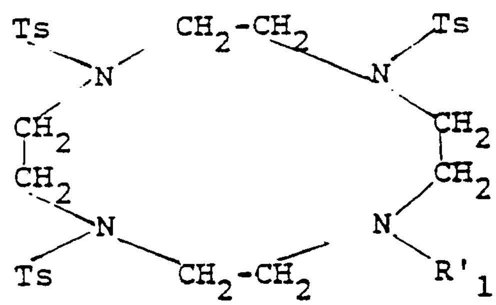

wherein the symbol "Ts" represents the tosyl (p-toluenesulfonyl) group. This general approach to polyazamacrocycles is described in Org. Synth., 58:86 (1978). The 4-substituted 1,4,7-triaza-heptanes can be prepared using the methodology described in United States patent 3,201,472.

Removal of the tosyl groups from a compound of formula IV yields the desired compounds of formula II wherein Y is

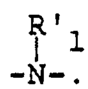

It can be accomplished by acid hydrolysis using, for example, concentrated sulfuric acid or hydrobromic acid with acetic acid and phenol or by reductive cleavage using, for example, lithium aluminium hydride or sodium in liquid ammonia.

Alternatively, the starting compounds of formula II wherein Y is

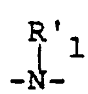

can be prepared by reducing the corresponding compound having the formula

V

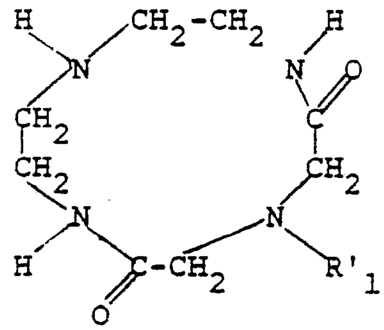

using phosphorous oxychloride or phosphorous pentachloride and zinc or sodium borohydride, lithium aluminium hydride, or borane. Compounds of formula V can be prepared by cyclocondensation of diethylenetriamine with diesters of substituted imino diacetic acids, i.e., compounds of the formula

VI

$$R'_1\text{-}N\text{-}(CH_2\text{-}\overset{\displaystyle O}{\overset{\|}{C}}\text{-}O\text{-}alkyl)_2 .$$

The following examples are specific embodiments of this invention.

## Example 2

### 1,4,7-Triscarboxymethyl-1,4,7,10-tetraazacyclododecane

#### Method I

A solution of 36.8 g (0.100 mol) 1,4,7,10-tetraazacyclododecane bissulfuric acid salt in 166 ml deionized water was brought to pH 8.5 using 6M potassium hydroxide. To this solution was added 18.9 g (0.200 mol) of solid chloroacetic acid, and the pH was readjusted to 8.5. The temperature was increased to 50 ° C and the pH maintained between 8.5-9.0 by the addition of 6M potassium hydroxide as necessary. After 3 hours, an additional 4.73 g (0.050 mol) of chloroacetic acid was added, and the pH readjusted. After 5 hours, an additional 3.78 g (0.040 mol) of chloroacetic acid was added and the pH was readjusted. The reaction was continued at 50 ° C and pH 8.5-9.0 for 16 hours after the second addition. The reaction mixture was cooled, the pH brought to 2 with concentrated hydrochloric acid, and the mixture diluted with methanol. The mixture was filtered and the filtrate evaporated. The solid was dissolved in water and passed onto a cation exchange column (Dowex 50X2-400, hydrogen form). The column was washed well with water then the ligand brought off by eluting with 0.5M ammonium hydroxide. Evaporation gave the solid ammonium salt. This salt was dissolved in water and passed onto a column of anion exchange resin (Dowex AG1-X8). The column was washed well with water and the ligand eluted with 0.5M aqueous formic acid. The solid obtained after evaporation of the solvent was crystallized from methanol to give 8.2 g of the ligand as a colorless solid. $^{13}C$ NMR ($D_2O$, ppm vs TMS): 176.9, 171.0, 57.0, 55.7, 52.7, 50.3, 49.3, 43.6. Mass spectrum (FAB): m/e 345 (M-H) and 347 (M+H).

#### Method II

A mixture of 100 mg of 10% palladium on charcoal and 250 mg of 1-benzyl-4,7,10-triscarboxymethyl-1,4,7,10-tetraazacyclododecane (see Example 4) in 40 ml of 5% acetic acid in water was shaken under 35.8 p.s.i. of hydrogen for 16 hours. Filtration and evaporation gave the crude ligand which was crystallized from methanol/acetone yielding 130 mg of the desired product.

## Example 3

### 1-Methyl-4,7,10-triscarboxymethyl-1,4,7,10-tetraazacyclododecane

To a solution of 250 mg (0.723 mmol) of 1,4,7-triscarboxymethyl-1,4,7,10-tetraazacyclododecane (see Example 2) in 2.9 ml of methanol was added 220 mg (1.59 mmol) of potassium carbonate. To the resulting mixture was added 308 mg (2.17 mmol, 3 equiv) of methyl iodide. Within a short time, most of the solids dissolved. After 15 hours at 21 °C, a mass of crystals had separated. Additional methanol was added (2 ml) to dissolve the solid. After 23 hours, an additional 102 mg (0.72 mmol) of methyl iodide was added. After an additional 16 hours, the solution was acidified with concentrated hydrochloric acid and the volatiles were removed on the rotary evaporator. The residue was extracted with methanol, filtered, and the methanol evaporated. The residue was crystallized twice from methanol/acetone yielding 56 mg (0.16 mmol) of a colorless solid, melting point 215-240° (dec.). $^{13}C$ NMR ($D_2O$, ppm vs TMS): 177.2, 171.1, 57.2, 56.5, 54.1, 52.6, 50.1, 49.9, 43.7. Mass spectrum (FAB): m/e 359 (M-H) and 361 (M+H).

Example 4

1-Benzyl-4,7,10-triscarboxymethyl-1,4,7,10-tetraazacyclododecane

A) 5-Benzyl-2,8-dioxo-1,5,9-triazanonane

To a solution of 6.80 g (95.7 mmol) acrylamide (95.7 mmol) in 10 ml water at ca. 5 °C was added dropwise 4.4 ml (4.32 g, 40.3 mmol) of benzyl amine. After the addition was complete, the temperature was raised to 87 °C for 6 hours. The water was evaporated to give a thick oil. The oil was dissolved in about 25 ml acetone and about 15 ml of ether was added to precipitate an oil. This mixture was allowed to stand for 16 hours, during which time the oil solidified. The solid was broken up and collected by filtration and dried under vacuum at 50 °C for 6 hours. The crude product weighed 9.75 g (39.1 mmol), melted at 103-106 °C, and was suitable for use in the next reaction without further purification.

B) 4-Benzyl-1,4,7-triazaheptane

To a solution of 38.7 g (0.586 mol) potassium hydroxide in 150 ml water at 5 °C was added 30.0 g (0.120 ml) of 5-benzyl-2,8-dioxo-1,5,9-triazanonane. To the resulting mixture was added dropwise 345 ml of 0.80M potassium hypochlorite over 0.5 hours. The solution was allowed to warm to 21 °C then heated to 85 °C for 4 hours. The solvent was then evaporated under reduced pressure and the residue was extracted with dichloromethane, filtered, dried with magnesium sulfate, filtered, and evaporated to give 14.7 g of the crude product. Vacuum distillation gave 10.1 g of the desired triamine as a colorless liquid, boiling point 110-115 °C at 0.35 mm. of Hg. $^{13}C$ NMR ($CDCl_3$, ppm vs TMS): 139.1, 128.7, 128.1, 126.9, 59.0, 56.3, 39.3. Mass spectrum (CI): m/e 194 (M+H) and 192 (M-H).

C) 4-Benzyl-1,7-bis(p-toluenesulfonyl)-1,4,7-triazaheptane

To a solution of 141.2 g (0.741 mol) of p-toluenesulfonyl chloride in 250 ml of dichloromethane with 110 ml (0.8 mol) of triethylamine was added dropwise 68.0 g (0.352 mol) of 4-benzyl-1,4,7-triazaheptane in 75 ml of dichloromethane. After 2 hours, the solution was washed three times with water at pH 9, and the organic phase was dried with sodium sulfate and filtered. Evaporation gave an oil which was dissolved in about 450 ml of ethyl acetate. The solution was diluted with 200 ml of ether and left to stand at room temperature for 24 hours. The mixture was further diluted with about 50 ml of ether and refrigerated for another day. The product crystallized in massive prisms which were collected by filtration and dried under vacuum at 40 °C for 6 hours, yielding in the first crop 140 g (0.279 mol) of a colorless solid; melting point 87-91 °C. Mass spectrum (CI): m/e 502 (M+H) and 500 (M-H).

D) 1-Benzyl-4,7,10-tris(p-toluenesulfonyl)-1,4,7,10-tetraazacyclododecane

Into a dry flask under nitrogen was palced about 3.9 g of a 60% sodium hydride dispersion. It was washed twice with hexanes then suspended in 200 ml of dry dimethylformamide. To the mixture was added 20 g (40 mmol) of 4-benzyl-1,7-bis(p-toluenesulfonyl)-1,4,7-triazaheptane over 5 minutes. After the initial reaction had subsided, the mixture was heated to 110 °C for 1 hour. To the resulting hot solution was added dropwise 22.6 g (40 mmol) of diethanolamine tritosylate in 100 ml of dry dimethylformamide over 3.5 hours. After an additional 0.5 hours, the solution was allowed to cool and 20 ml of methanol was added. The volatiles were then removed on the rotary evaporator. The residue was dissolved in a mixture of 400 ml of water and 200 ml of dichloromethane. The phases were separated and the aqueous phase washed twice

more with dichloromethane. The combined organic fractions were dried (magnesium sulfate), filtered, and evaporated to give a yellow oil. Crystallization was induced by the addition of about 100 ml of methanol. The mixture was kept at -5° C overnight and the product collected by filtration. After drying, 20.4 g of a colorless solid was obtained; melting point 208-210° C.

E) 1-Benzyl-1,4,7,10-tetraazacyclododecane tetrahydrochloride

Method I

To a slurry of 2.0 g (2.8 mmol) 1-benzyl-4,7,10-tris(p-toluenesulfonyl)-1,4,7,10-tetraazacyclododecane in about 25 ml of ammonia at -77° C under nitrogen was added 0.50 g (22 mmol, 8 equiv) of sodium metal in portions over about 5 minutes. The blue mixture was stirred an additional 45 minutes and the reaction was quenched with 1.16 g (22 mmol) of solid ammonium chloride. The ammonia was allowed to evaporate. Water (50 ml) was added to the residue and the pH adjusted to about 12 using 6M potassium hydroxide. The mixture was extracted three times with 30 ml portions of dichloromethane. The combined organic fractions were then extracted with three 30 ml portions of 2M hydrochloric acid. Evaporation of the water under reduced pressure gave a solid residue. The residue was washed with methanol and dried under vacuum at 50° C to give 600 mg (1.47 mmol) of a colorless solid, which was used directly in the final step.

Method II

1. 1-Benzyl-3,11-dioxo-1,4,7,10-tetraazacyclododecane

To a solution of 31.2 g dimethyl-N-benzyl-iminodiacetate in 2.5 l of dry ethanol at reflux under nitrogen was added dropwise 12.8 g of diethylenetriamine in 160 ml of dry ethanol. Reflux was carried out for a total of 137 hours. The solution was evaporated under reduced pressure leaving a yellow paste. Trituration with acetone left 5.2 g of the desired product as a colorless solid.
$^{13}C$ NMR (methanol, ppm vs TMS): 173.6, 139.0, 130.5, 129.6, 128.8, 64.0, 46.3, 38.7. Mass spectrum (CI): m/e 291 (M+H) and 289 (M-H).

2. 1-Benzyl-1,4,7,10-tetraazacyclododecane

To a suspension of 890 mg (3.07 mmol) of 1-benzyl-3,11-dioxo-1,4,7,10-tetraazacyclododecane in tetrahydrofuran under nitrogen was added 2.64 ml of 8M borane-methyl sulfide complex (21.1 mol, 7 equivalents). The mixture was heated to reflux allowing the methyl sulfide to distill out of the reaction flask. After 2 hours, the reaction was quenched by the addition of 12 ml 1.8M hydrochloric acid in methanol and refluxed for an additional 3 hours. Volatiles were removed by evaporation, and the solid resuspended in methanol and reevaporated. The product was crystallized from methanol/ethyl acetate; yield 455 mg, 37%. Mass spectrum (CI): m/e 263 (M+H).

F) 1-Benzyl-4,7,10-triscarboxymethyl-1,4,7,10-tetraazacyclododecane

The pH of a solution of 3.5 g of 1-benzyl-1,4,7,10-tetraazacyclododecane tetrahydrochloride in 17 ml of water was adjusted to 7 using 6.0 M potassium hydroxide. To this solution was added 3.64 g of chloroacetic acid, and the pH was readjusted to 9.5. The solution was warmed to 45° C and the pH adjusted as necessary to maintain the pH at 9.5-10. After 6 hours, the heat source was removed and the solution left to stand for 1 day. The solution was acidified to pH 3 with concentrated hydrochloric acid, diluted with 500 ml of water, and applied to a Dowex 50X-2 cation exchange resin ($H^+$ form). After washing with water, the ligand was eluted with 0.5 M aqueous ammonia. After evaporation of the solvents, the crude ammonium salt was redissolved in water and applied to an anion exchange column. After washing with water, the ligand was eluted with 0.2 m aqueous formic acid. After evaporation of the solvents, the crude product was crystallized from methanol/acetone to give 2.0 g of the ligand as a colorless solid. Mass spectrum (FAB): m/e 437 (M+H) and 435 (M-H).

Example 5

Gadolinium(III)(1,4,7-triscarboxymethyl-1,4,7,10-tetraazacyclododecane)

Method I

To a solution of 9.05 g (26.1 mmol) of 1,4,7-triscarboxymethyl-1,4,7,10-tetraazacyclododecane (see Example 2) in 50 ml of water was added 4.74 g (13.1 mmol) of solid gadolinium oxide. The mixture was heated to 90 °C for 4 hours, during which time most of the solid dissolved. The mixture was filtered and the filtrate evaporated to dryness under reduced pressure. The gummy residue was twice dissolved in ethanol and evaporated to dryness. The colorless solid residue was dissolved in nitromethane, filtered through a fine porosity sintered glass funnel, and the filtrate placed in a flask in a closed container also holding about 1 liter of water. Diffusion of water into the organic solution over several days gave a colorless solid precipitate. The precipitate was collected by filtration, washed with nitromethane, resuspended in acetone and washed well with that solvent, then dried under vacuum at 60 °C for 2 days yielding 10.7 g of a colorless solid.
Anal. Calcd. for 90.06% ligand, 9.94% water; C, 30.25; H, 5.28; N, 10.08.
Found: C, 30.25; H, 5.48; N, 9.97; C/N = 14.4

Method II

Gadolinium acetate tetrahydrate (145.5 mg) was dissolved in 3 ml of deionized water. Aqueous 1M 1,4,7-triscarboxymethyl-1,4,7,10-tetraazacyclododecane was added to the gadolinium acetate solution, mixed and adjusted to pH 3. The mixture was heated for 20 minutes at 88 °C and adjusted to pH 7.3 with 1N sodium hydroxide. The free gadolinium content was measured by paper thin layer chromatograhy. Twice the quantity of 1,4,7-triscarboxymethyl-1,4,7,10-tetraazacyclododecane required to chelate any free gadolinium was added. The solution was adjusted to pH 3, heated at 88 °C for 20 minutes and then adjusted to pH 7.3. Free gadolinium content was determined and 1,4,7-triscarboxymethyl-1,4,7,10-tetraazacyclododecane was added as required. The sample was adjusted to 7 ml with deionized water, passed through a 0.22 μ filter (Millipore) into a vial, stoppered and sealed.

Example 7

$^{99m}$Technetium(1,4,7-triscarboxymethyl-1,4,7,10-tetraazacyclododecane)

100mM of calcium (II) (1,4,7-triscarboxymethyl-1,4,7,10-tetraazacyclododecane) was prepared by mixing equal volumes of 200mM of calcium chloride and 200mM of 1,4,7-triscarboxymethyl-1,4,7,10-tetraazacyclododecane. One and one-half ml of the solution was adjusted to pH 8.8 with dilute sodium hydroxide and 150 μl of 0.88% stannous chloride was added and mixed. Technetium-99m was added to obtain a final concentration of 20 μCi/ml and the solution was adjusted to pH 3. The solution was heated at 88 °C for 20 minutes, cooled, and adjusted to pH 7. After adjusting to a volumne of 3 ml, it was passed through a 0.22 μ filter.

Example 8

Gallium(III)(1,4,7-triscarboxymethyl-1,4,7,10-tetraazacyclododecane)

(1,4,7-Triscarboxymethyl-1,4,7,10-tetraazacyclododecane (69.3 mg) and 58.8 mg of dihydrated calcium chloride were mixed in water to yield calcium (II) (1,4,7-triscarboxymethyl-1,4,7,10-tetraazacyclododecane). 90 μCi of $^{67}$gallium was added. The solution was adjusted to pH 3, heated at 88 °C for 20 minutes and adjusted to pH 7.3.

Example 9

Bismuth(III)(1,4,7-triscarboxymethyl-1,4,7,10-tetraazacyclododecane)

50mM Bismuth(III) (1,4,7-triscarboxymethyl-1,4,7,10-tetraazacyclododecane) was prepared by combining 24.2 mg of bismuth nitrate with 100μl of 1M 1,4,7-triscarboxymethyl-1,4,7,10-tetraazacyclododecane and 140 μl of water. The solution was adjusted to pH 3 with dilute sodium hydroxide. The mixture was heated at 88 °C until the bismuth nitrate dissolved (ca. 30 minutes). The solution was adjusted to pH 7.3 with dilute sodium hydroxide and reheated briefly at 88 °C until a small quantity of precipitate was dissolved. On cooling, the solution remained clear.

Determination of free bismuth by precipitation and x-ray fluorescence spectroscopy showed that >99% of the bismuth had been chelated.

Example 10

Chromium, Iron, Manganese and Dysprosium Chelates of (1,4,7-triscarboxymethyl-1,4,7,10-tetraazacyclododecane)

Four hundred fifty µl of 100mM solutions of each of chromic chloride, ferric chloride, manganese chloride and dysprosium chloride were mixed with 50 µl of 1M 1,4,7-triscarboxymethyl-1,4,7,10-tetraazacyclododecane and adjusted to pH 4.5. The solutions were heated at 88°C for 20 minutes to enhance the rate of chelation, cooled and then adjusted to pH 7.

To determine if chelation had occurred, the solutions were diluted to a concentration of 1mM metal chelate. An aliquot was tested by measuring its relaxivity and comparing it with the relaxivity of the metal ion alone. The data demonstrated clearly that the metal ions had been chelated. The relaxivity is proportional to the number of water molecules bound to the metal. The chelator displaces coordinated water molecules and thus lowers the relaxivity. Relaxitives of metal chelates are shown in the following table.

Relaxivities of Metal Chelates at 20MHz

| Chelate Metal | $K_1$ ($Mole^{-1}$ $Sec.^{-1}$) |
|---|---|
| Dysprosium(III) (1,4,7-triscarboxymethyl-1,4,7,10-tetraazacyclododecane) | 177 |
| Dysprosium chloride | 525 |
| Iron(III)(1,4,7-triscarboxymethyl-1,4,7,10-tetraazacyclododecane) | 530 |
| Ferric chloride | 3374 |
| Chromium(III)(1,4,7-triscarboxymethyl-1,4,7,10-tetraazacyclododecane) | 422 |
| Chromic chloride | 3270 |
| Manganese(III)(1,4,7-triscarboxymethyl-1,4,7,10-tetraazacyclododecane)(sodium salt) | 1151 |
| Manganese chloride | 6250 |

Example 11

Gadolinium(III)(4,7,10-triscarboxymethyl-1-methyl-1,4,7,10-tetraazacyclododecane)

Gadolinium acetate tetrahydrate (102 mg) was mixed with 133 mg of 4,7,10-triscarboxymethyl-1-methyl-1,4,7,10-tetraazacyclododecane. To the mixture was added 250 µCi of $^{153}$gadolinium nitrate. The solution was adjusted to pH 3 with 1N hydrochloric acid and heated for 20 minutes at 88°C. The solution was adjusted to pH 7. Free gadolinium content was 5.07%. Additional ligand, 36 mg, was added, the solution was adjusted to pH 3 and heated as before. The solution was adjusted to pH 7.3 and tested by the thin layer chromatography procedure. Free gadolinium content was 0.14%.

## Claims

**1.** A compound having the formula

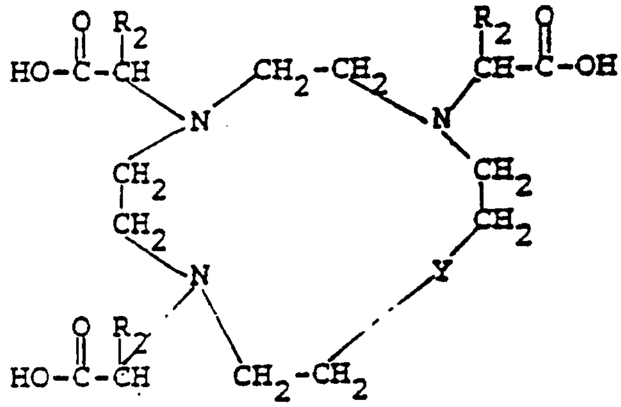

or a salt thereof, wherein Y is

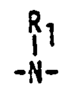

$R_1$ is hydrogen, alkyl, arylalkyl or aryl, $R_2$ is hydrogen or alkyl and wherein aryl refers to phenyl and phenyl substituted with 1, 2 or 3 halogen, hydroxyl, alkyl, alkoxy, carbamoyl or carboxyl groups and furthermore wherein alkyl and alkoxy refer to both straight and branched chain $C_1$ to $C_5$ groups.

**2.** A compound in accordance with claim 1 wherein Y is

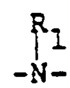

and $R_1$ is hydrogen.

**3.** A compound in accordance with claim 1 wherein Y is

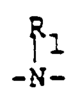

and $R_1$ is alkyl.

**4.** A compound in accordance with claim 3 wherein Y is

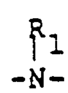

and $R_1$ is methyl.

**5.** A compound in accordance with claim 1 wherein Y is

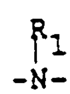

and $R_1$ is arylalkyl.

6. A compound in accordance with claim 1 wherein Y is

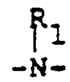

and $R_1$ is benzyl.

7. A compound in accordance with claim 1 wherein Y is

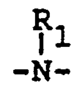

and $R_1$ is aryl.

8. A compound in accordance with claim 1 wherein $R_2$ is hydrogen.

9. A compound in accordance with claim 1 wherein $R_2$ is alkyl.

10. A compound in accordance with claim 9 wherein $R_2$ is methyl.

11. The compound in accordance with claim 1, 1,4,7-triscarboxymethyl-1,4,7,10-tetraazacyclododecane.

12. The compound in accordance with claim 1, 1-methyl-4,7,10-triscarboxymethyl-1,4,7,10-tetraazacyclododecane.

13. The compound in accordance with claim 1, 1-benzyl-4,7,10-triscarboxymethyl-1,4,7,10-tetraazacyclododecane.

14. A complex, or a salt of a complex, of a metal-chelating ligand having the formula

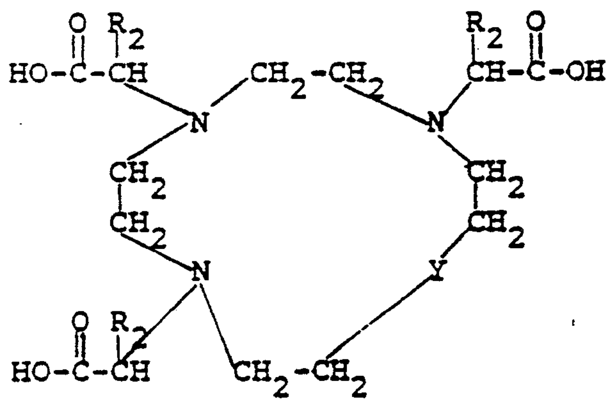

wherein Y is

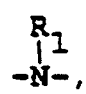

$R_1$ is hydrogen, alkyl, arylalkyl or aryl, and $R_2$ is hydrogen or alkyl and a paramagnetic metal atom of low toxicity and wherein aryl refers to phenyl and phenyl substituted with 1, 2 or 3 halogen, hydroxyl, alkyl, alkoxy, carbamoyl or carboxyl groups and furthermore wherein alkyl and alkoxy refer to both

straight and branched chain $C_1$ to $C_5$ groups.

**15.** A complex in accordance with claim 14 wherein the paramagnetic metal atom is gadolinium, manganese, chromium or iron.

**16.** A complex in accordance with claim 14 wherein the metal chelating ligand is 1,4,7-triscarboxymethyl-1,4,7,10-tetraazacyclododecane.

**17.** A complex in accordance with claim 14 wherein the metal chelating ligand is 1-methyl-4,7,10-triscarboxymethyl-1,4,7,10-tetraazacyclododecane.

**18.** A complex in accordance with claim 14 wherein the chelating ligand is 1-benzyl-4,7,10-triscarboxymethyl-1,4,7,10-tetraazacyclododecane.

**19.** A compound having the formula

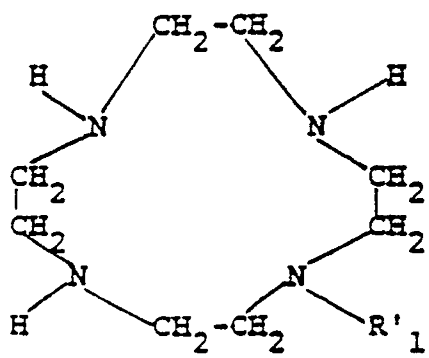

wherein $R'_1$ is alkyl, arylalkyl or aryl, and wherein aryl refers to phenyl and phenyl substituted with 1, 2 or 3 halogen, hydroxyl, alkyl, alkoxy, carbamoyl or carboxyl groups and furthermore wherein alkyl and alkoxy refer to both straight and branched chain $C_1$ to $C_5$ groups.

## Revendications

**1.** Composé de formule

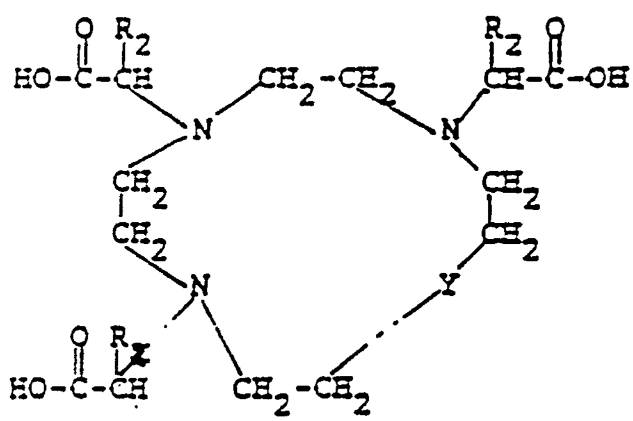

éventuellement sous forme de sel, formule dans laquelle Y est

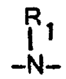

où $R_1$ est l'hydrogène ou un radical alkyle, arylalkyle ou aryle, $R_2$ est l'hydrogène ou un radical alkyle, le radical aryle étant un radical phényle éventuellement substitué par 1, 2 ou 3 atomes d'halogène ou groupements hydroxyle, alkyle, alcoxy, carbamoyle ou carboxyle, et les radicaux alkyle et alcoxy étant des radicaux en $C_1$ à $c_5$ à chaîne droite ou ramifiée.

**2.** Composé selon la revendication 1, dans lequel Y est

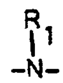

et $R_1$ est l'hydrogène.

**3.** Composé selon la revendication 1, dans lequel Y est

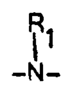

et $R_1$ est un radical alkyle.

**4.** Composé selon la revendication 3, dans lequel Y est

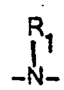

et $R_1$ est le radical méthyle.

**5.** Composé selon la revendication 1, dans lequel Y est

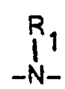

et $R_1$ est un radical arylalkyle.

**6.** Composé selon la revendication 1, dans lequel Y est

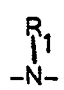

et $R_1$ est le radical benzyle.

**7.** Composé selon la revendication 1, dans lequel Y est

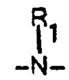

et $R_1$ est un radical aryle.

**8.** Composé selon la revendication 1, dans lequel $R_2$ est l'hydrogène.

**9.** Composé selon la revendication 1, dans lequel $R_2$ est un radical alkyle.

**10.** Composé selon la revendication 9, dans lequel $R_2$ est le radical méthyle.

**11.** Composé selon la revendication 1, qui est le 1,4,7-triscarboxyméthyl-1,4,7,10-tétraazacyclododécane.

**12.** Composé selon la revendication 1, qui est le 1-méthyl-4,7,10-triscarboxyméthyl-1,4,7,10-tétraazacyclododécane.

**13.** Composé selon la revendication 1, qui est le 1-benzyl-4,7,10-triscarboxyméthyl-1,4,7,10-tétraazacyclododécane.

**14.** Complexe, ou sel de complexe, d'un coordinat chélateur de métaux ayant pour formule

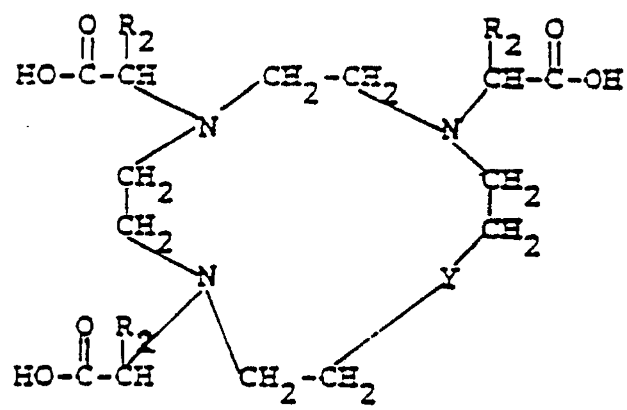

dans laquelle Y est

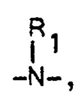

$R_1$ est l'hydrogène ou un radical alkyle, arylalkyle ou aryle, et $R_2$ est l'hydrogène ou un radical alkyle, et d'un atome de métal paramagnétique de faible toxicité, le radical aryle étant un radical phényle éventuellement substitué par 1, 2 ou 3 atomes d'halogène ou groupements hydroxy, alkyle, alcoxy, carbamoyle ou carboxyle, et les radicaux alkyle et alcoxy étant des radicaux en $C_1$ à $C_5$ à chaîne droite ou ramifiée.

**15.** Complexe selon la revendication 14, dans lequel l'atome de métal paramagnétique est un atome de gadolinium, de manganèse, de chrome ou de fer.

**16.** Complexe selon la revendication 14, dans lequel le coordinat chélateur de métaux est le 1,4,7-triscarboxyméthyl-1,4,7,10-tétraazacyclododécane.

**17.** Complexe selon la revendication 14, dans lequel le coordinat chélateur de métaux est le 1-méthyl-4,7,10-triscarboxyméthyl-1,4,7,10-tétraazacyclododécane.

**18.** Complexe selon la revendication 14, dans lequel le coordinat chélateur de métaux est le 1-benzyl-4,7,10-triscarboxyméthyl-1,4,7,10-tétraazacyclododécane.

**19.** Composé de formule

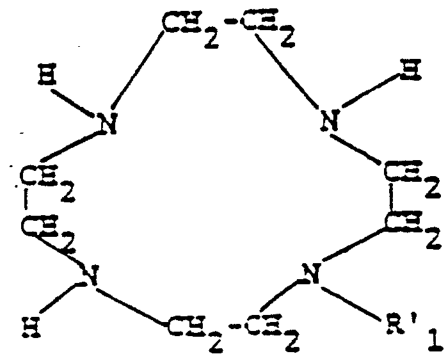

dans laquelle $R'_1$ est un radical alkyle, arylalkyle ou aryle, le radical aryle étant un radical phényle éventuellement substitué par 1, 2 ou 3 atomes d'halogène ou groupements hydroxyle, alkyle, alcoxy, carbamoyle ou carboxyle, et les radicaux alkyle et alcoxy étant des radicaux en $C_1$ à $C_5$ à chaîne droite ou ramifiée.

**Patentansprüche**

**1.** Verbindung der Formel

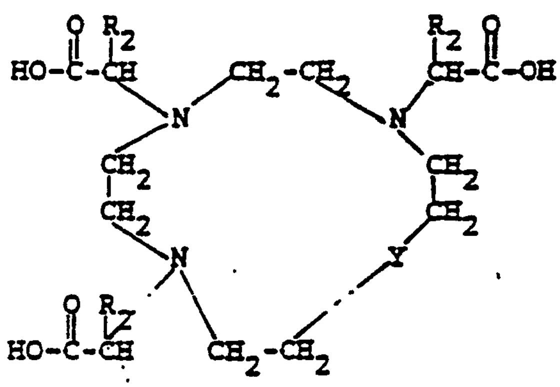

oder ein Salz davon, in der Y

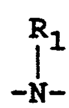

bedeutet,

$R_1$ ein Wasserstoffatom, einen Alkyl-, Arylalkyl- oder Arlyrest bedeutet, $R_2$ ein Wasserstoffatom oder einen Alkylrest darstellt, wobei sich Aryl auf Phenyl und mit 1, 2 oder 3 Halogenatomen, Hydroxy-, Alkyl-, Alkoxy-, Carbamoyl- oder Carboxylgruppen substituierte Phenylgruppen bezieht und wobei sich außerdem Alkyl und Alkoxy sowohl auf unverzweigte als auch auf verzweigtkettige $C_1$ bis $C_5$-Reste bezieht.

**2.** Verbindung nach Anspruch 1, in der Y den Rest

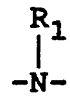

bedeutet und $R_1$ ein Wasserstoffatom ist.

**3.** Verbindung nach Anspruch 1, in der Y den Rest

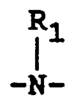

bedeutet und $R_1$ ein Alkylrest ist.

**4.** Verbindung nach Anspruch 1, in der Y den Rest

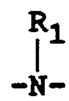

bedeutet und $R_1$ eine Methylgruppe ist.

**5.** Verbindung nach Anspruch 1, in der Y den Rest

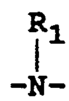

bedeutet und $R_1$ einen Arylalkylrest bedeutet.

**6.** Verbindung nach Anspruch 1, in der Y den Rest

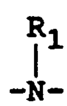

bedeutet und $R_1$ eine Benzylgruppe ist.

**7.** Verbindung nach Anspruch 1, in der Y den Rest

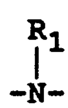

bedeutet und $R_1$ ein Arylrest ist.

**8.** Verbindung nach Anspruch 1, in der $R_2$ ein Wasserstoffatom ist.

**9.** Verbindung nach Anspruch 1, in der $R_2$ ein Alkylrest ist.

**10.** Verbindung nach Anspruch 9, in der $R_2$ eine Methylgruppe ist.

**11.** Verbindung nach Anspruch 1, nämlich 1,4,7-Triscarboxymethyl-1,4,7,10-tetraazacyclododecan.

**12.** Verbindung nach Anspruch 1, nämlich 1-Methyl-4,7,10-triscarboxymethyl-1,4,7,10-tetraazacyclododecan.

**13.** Verbindung nach Anspruch 1, nämlich 1-Benzyl-4,7,10-triscarboxymethyl-1,4,7,10-tetrazacyclododecan.

**14.** Ein Komplex oder ein Salz eines Komplexes eines Metallchelatisierenden Liganden mit der Formel

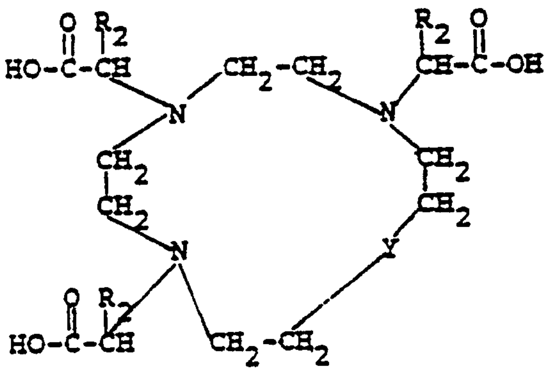

in der Y

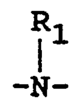

bedeutet, $R_1$ ein Wasserstoffatom, einen Alkyl-, Arylalkyl- oder Arylrest bedeutet und $R_2$ ein Wasserstoffatom oder einen Alkylrest darstellt, und einem paramagnetischem Metallatom niedriger Toxizität, wobei sich Aryl auf Phenyl- und mit 1, 2 oder 3 Halogenatomen, Hydroxy-, Alkyl-, Alkoxy-, Carbamoyl- oder Carboxylgruppen substituierte Phenylgruppen bezieht und wobei sich ferner Alkyl und Alkoxy sowohl auf unverzweigte als auch auf verzweigtkettige $C_1$ bis $C_5$-Reste bezieht.

**15.** Komplex nach Anspruch 14, wobei das paramagnetische Metallatom Gadolinium, Mangan, Chrom oder Eisen ist.

**16.** Komplex nach Anspruch 14, wobei der Metall-chelatisierende Ligand 1,4,7-Triscarboxymethyl-1,4,7,10-tetraazacyclododecan ist.

**17.** Komplex nach Anspruch 14, wobei der Metall-chelatisierende Ligand 1-Methyl-4,7,10-triscarboxymethyl-1,4,7,10-tetraazacyclododecan ist.

**18.** Komplex nach Anspruch 14, wobei der chelatisierende Ligand 1-Benzyl-4,7,10-triscarboxymethyl-1,4,7,10-tetraazacyclododecan ist.

**19.** Verbindung der Formel

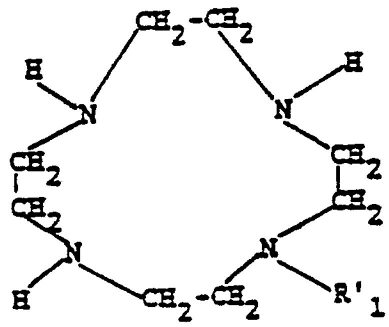

in der $R'_1$ einen Alkyl-, Arylalkyl- oder Arylrest bedeutet, wobei sich Aryl auf Phenyl- und mit 1, 2 oder 3 Halogenatomen, Hydroxyl-, Alkyl-, Alkoxy-, Carbamoyl- oder Carboxylgruppen substituierte Phenylgruppen bezieht und wobei sich ferner Alkyl und Alkoxy sowohl auf unverzweigte als auch auf verzweigtkettige $C_1$ bis $C_5$-Reste bezieht.